# EUROPEAN PATENT APPLICATION

(11) **EP 3 988 154 A1**
(43) Date of publication of application: **27.04.2022**
(21) Application number: 21203304.7
(22) Date of filing: 18.10.2021
(51) Int. Cl.: A61M 16/08, A61M 16/20, A61M 16/06, A61M 39/24, A61M 39/10

(54) **INTEGRATED MANIFOLD SYSTEM**

(30) Priority: 20.10.2020 US 202017074903
(71) Applicant: Avon Protection Systems, Inc., Cadillac, MI 49601 (US)
(72) Inventor: MAYHUE, Clinton Cain, Cadillac, 49601 (US); WILCOX, James, Cadillac, 49601 (US); MARTIN, Joseph Craig, Cadillac, 49601 (US)
(74) Representative: Mewburn Ellis LLP

(57) **Abstract**

An integrated manifold system is disclosed that combines in a single, compact, unitary assembly a one-piece, machined manifold (110, 210) for connecting air supply cylinders with a first stage regulator (120, 220) and recharge port (30). The manifold body (110, 210) has at least one bottle port (112, 212) configured to removably receive a bottle of pressurized air, a first stage regulator chamber (114, 214), a quick disconnect fitting port (115, 215), an air channel (113, 213) in fluid communication with the at least one bottle port and the first stage regulator chamber, an outlet channel (118, 218) in fluid communication with the first stage regulator chamber, and an on/off valve (170, 270) operatively positioned on a side of the manifold body (110, 210).

## Description

### GOVERNMENT INTEREST

This invention was made with government support under Contract No. N41756-17-C-4721 awarded by the U.S. Department of Defense. The government has certain rights in the invention.

### CROSS-REFERENCE TO RELATED APPLICATION

This application is a continuation-in-part of U.S. Application No. 16/137,847 titled "Integrated Manifold System," filed September 21, 2018, which claims the benefit of U.S. Provisional Application No. 62/563,714 titled "Integrated Manifold System," filed September 27, 2017, which applications are incorporated herein by reference in their entireties.

### FIELD OF THE INVENTION

This invention relates to systems and devices for the pressure-regulated supply of gases, and more particularly to a system and device providing a pressure-regulated supply of pressurized, breathable air, oxygen, or other gases in a compact, light-weight assembly.

### BACKGROUND OF THE INVENTION

First responders, members of the military, emergency personnel, and others are frequently confronted with inhospitable breathing environments, such as may occur in the event of fire, a hazardous chemical spill, a nuclear, chemical, or biological attack, or the like. In order to allow such personnel to accomplish their mission in such environment, various systems have been developed to provide the individual with personal breathing apparatus that ensures them of a clean, safe breathing environment, even when operating in the hostile environment. However, such operators are often placed under significant physical and other stress when operating in those environments, and are often confronted with small or obstructing spaces in which to operate. While previously known systems have been able to provide the operators secure breathing environments, their bulky size has made operation in highly stressful and/or obstructive environments quite challenging. Thus, there remains a need in the art to provide a system and apparatus that can reliably provide a safe, secure breathing environment but that minimizes the overall profile (i.e., physical size and weight) of the system so as to ease the stress on the operator during use.

### SUMMARY OF THE INVENTION

Disclosed herein is an integrated manifold system, usable for instance in self-contained breathing applications, that combines in a single, compact, unitary assembly a one-piece, machined manifold for connecting air supply cylinders with a first stage regulator and recharge port, providing a significant improvement over previously known systems, as it reduces potential points of failure in the system, reduces overall weight of the system, and eases the burden on the operator by not requiring them to carry and keep track of multiple, separate components for these features. While the exemplary embodiment described herein is principally with reference to the delivery of breathable air to an operator in an environmentally hazardous application, the system may likewise be used, by way of non-limiting example, to provide pressure-regulated delivery of high pressure, pure oxygen, such as might be desirable in medical procedures for delivering oxygen to a patient, in exothermic breaching applications using exothermic torch devices that employ oxygen, and such other applications that my require the portable delivery of pressure-regulated gases as will occur to those skilled in the art.

In accordance with certain aspects of an embodiment of the invention, an air supply system is disclosed comprising: a one-piece manifold body having at least one bottle port configured to removably receive a bottle of pressurized air, a first stage regulator chamber, a quick disconnect fitting port, an air channel in fluid communication with the at least one bottle port and the first stage regulator chamber, and an outlet channel in fluid communication with the first stage regulator chamber.

In accordance with further aspects of an embodiment of the invention, an air supply system is disclosed comprising: a one-piece manifold body having at least one bottle port configured to removably receive a bottle of pressurized air, a first stage regulator chamber, a quick disconnect fitting port, an air channel in fluid communication with the at least one bottle port and the first stage regulator chamber, and an outlet channel in fluid communication with the first stage regulator chamber; a first stage regulator operatively engaging the first stage regulator chamber; a mask hose quick disconnect in fluid communication with the outlet channel and configured to removably receive an air hose; and an on/off valve operatively engaging the air channel to regulate air flow between the at least one bottle port and the first stage regulator.

### BRIEF DESCRIPTION OF THE DRAWINGS

The accompanying drawings, which are included to provide further understanding of the invention and are incorporated in and constitute a part of this specification, illustrate embodiments of the present invention and together with the below description, serve to explain the principles of the invention.
FIG. 1 illustrates a front view of a prior art manifold for attaching air cylinders with the separate elements of a first stage regulator and recharge assembly.
FIG. 2 illustrates a perspective view of an integrated manifold system in accordance with certain aspects of an embodiment of the invention.
FIG. 3 illustrates a front view of the integrated manifold system of FIG. 2.
FIG. 4 illustrates a back view of the integrated manifold system of FIG. 2.
FIG. 5 illustrates a top view of the integrated manifold system of FIG. 2.
FIG. 6 is a cross-sectional view of the integrated manifold system of FIG. 5 along section line B-B.
FIG. 7 is a cross-sectional view of the integrated manifold system of FIG. 5 along section line A-A.

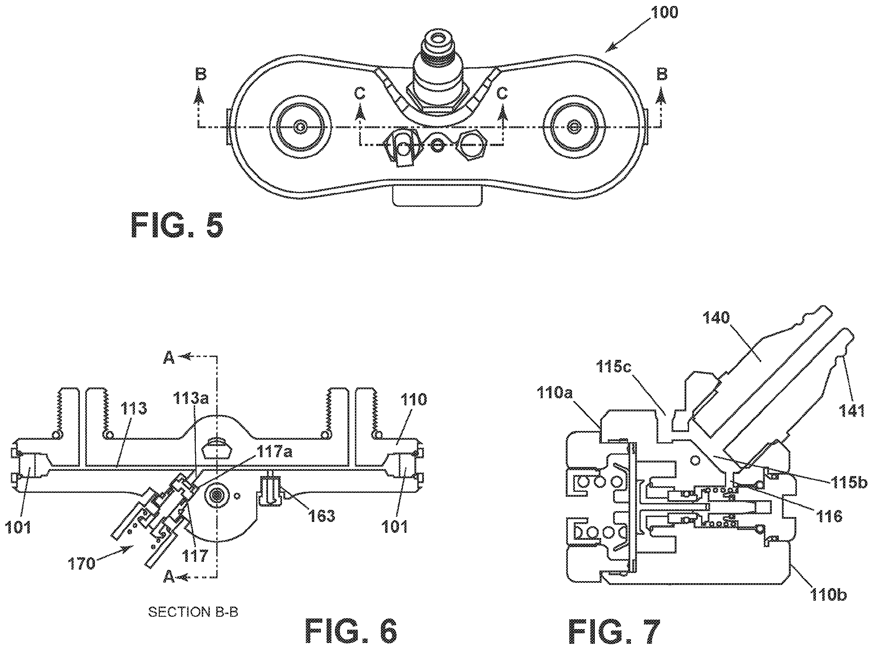

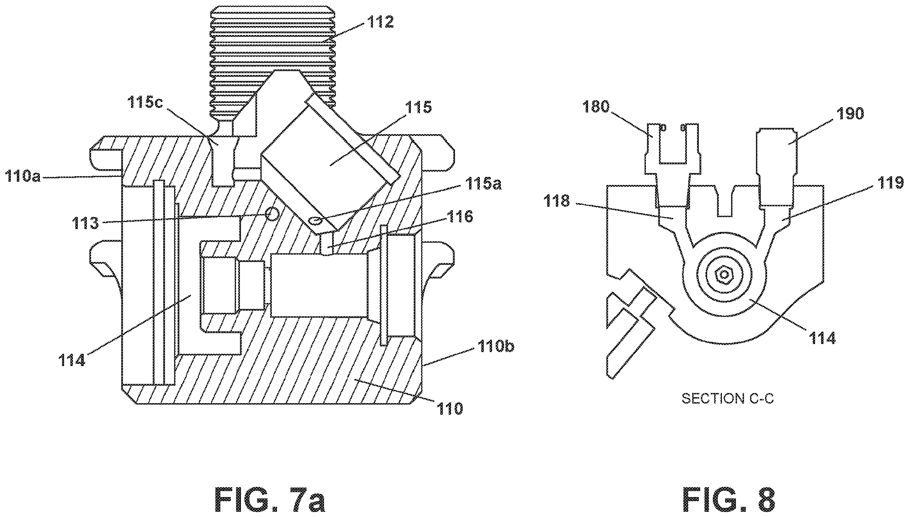

FIG. 7*a* is a cross-sectional view of only the manifold body of FIG. 5 along section line A-A.
FIG. 8 is a cross-sectional view of the integrated manifold system of FIG. 5 along section line C-C.

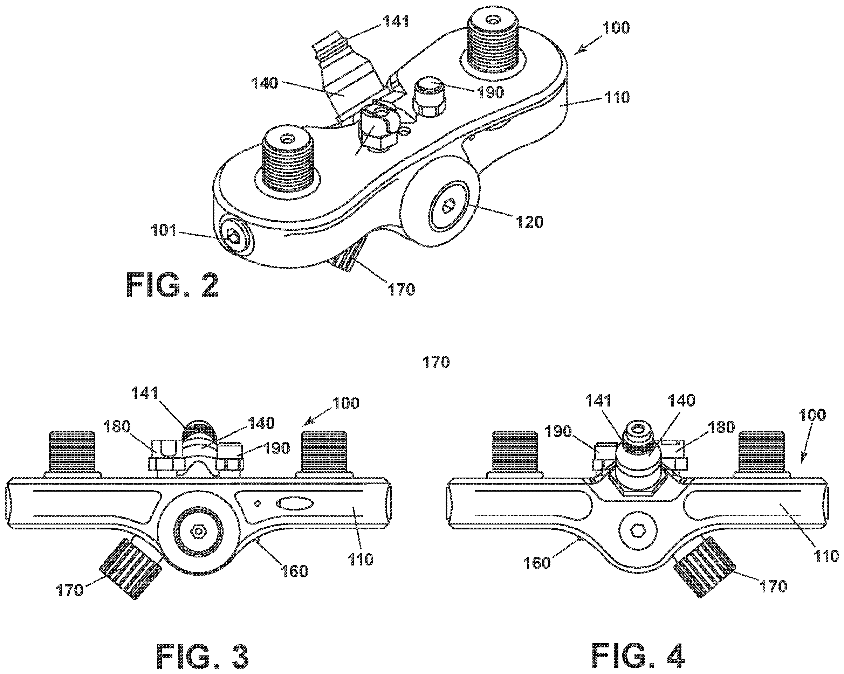

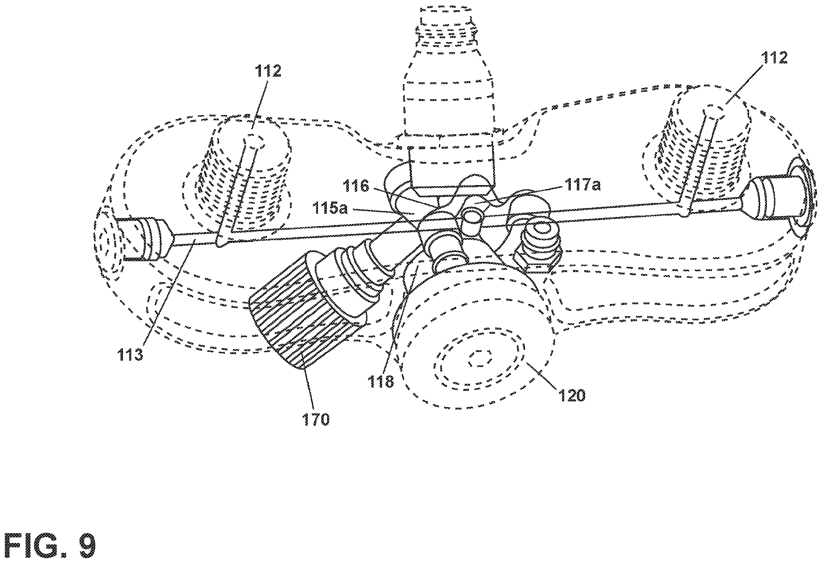

FIG. 9 is a perspective view of the integrated manifold system of FIG. 2 with the manifold body shown in phantom.

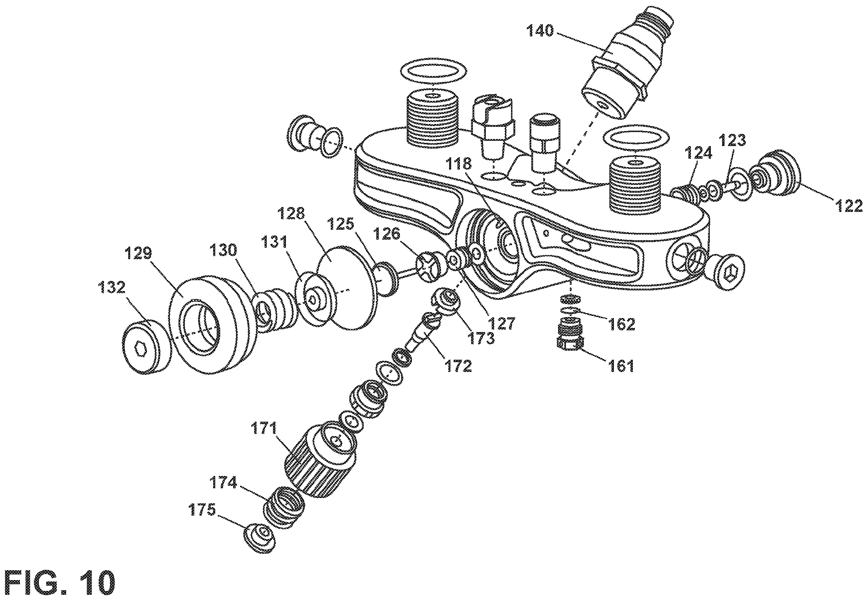

FIG. 10 is an exploded view of the integrated manifold system of FIG. 2.

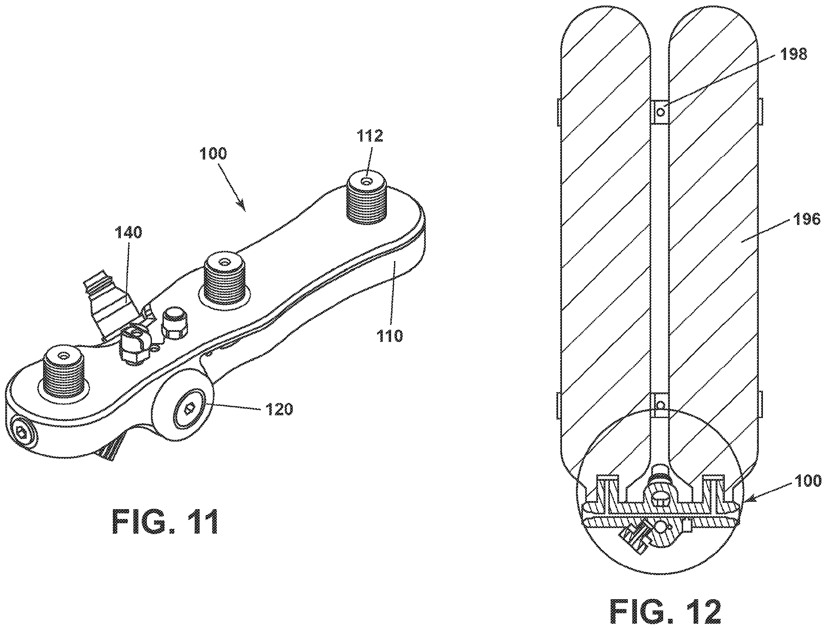

FIG. 11 is a perspective view of an integrated manifold system in accordance with further aspects of an embodiment of the invention.
FIG. 12 is a front, cross-sectional view of the manifold system of FIG. 2 including air bottles attached to the manifold body.

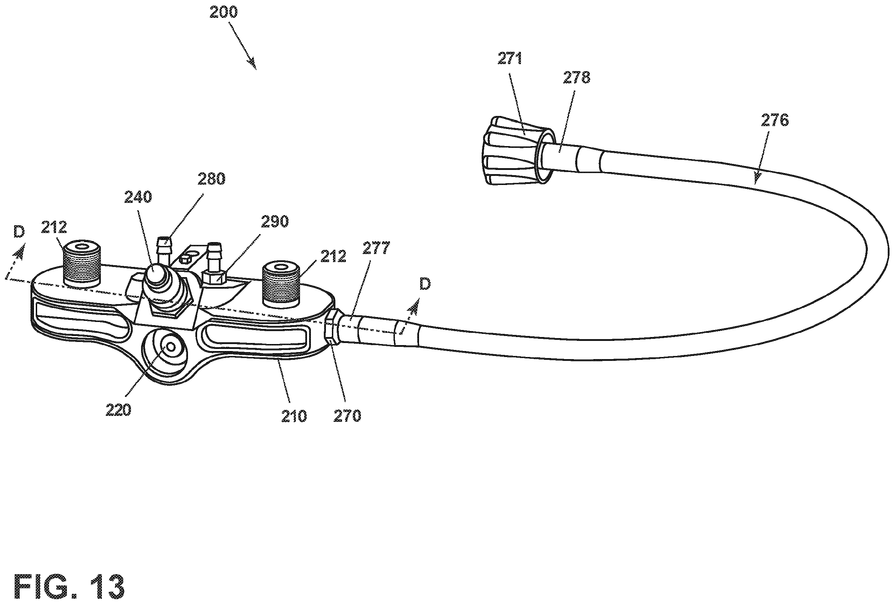

FIG. 13 is a perspective view of another integrated manifold system in accordance with various aspects described herein.

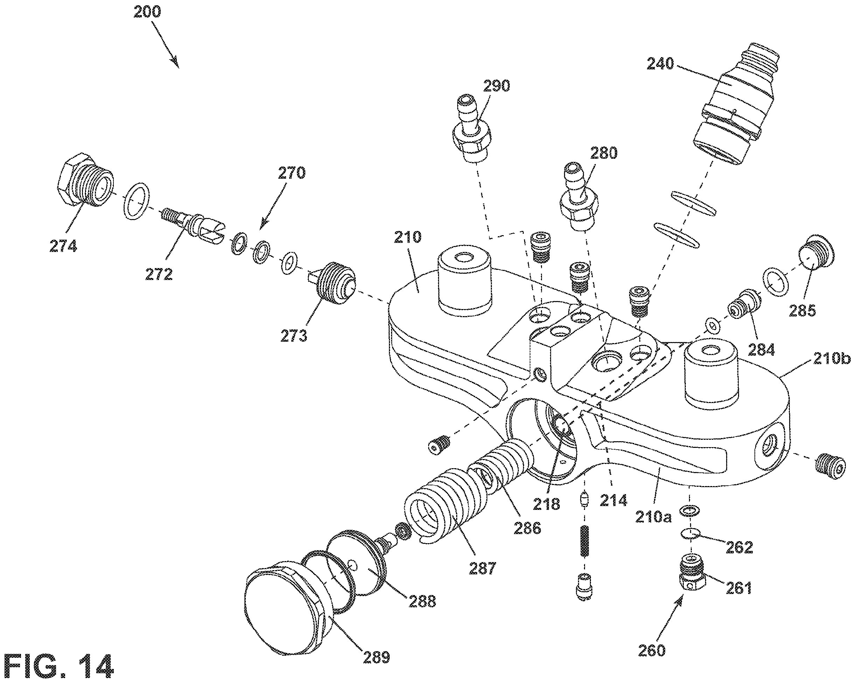

FIG. 14 is an exploded perspective view of a manifold body in the integrated manifold system of FIG. 13.

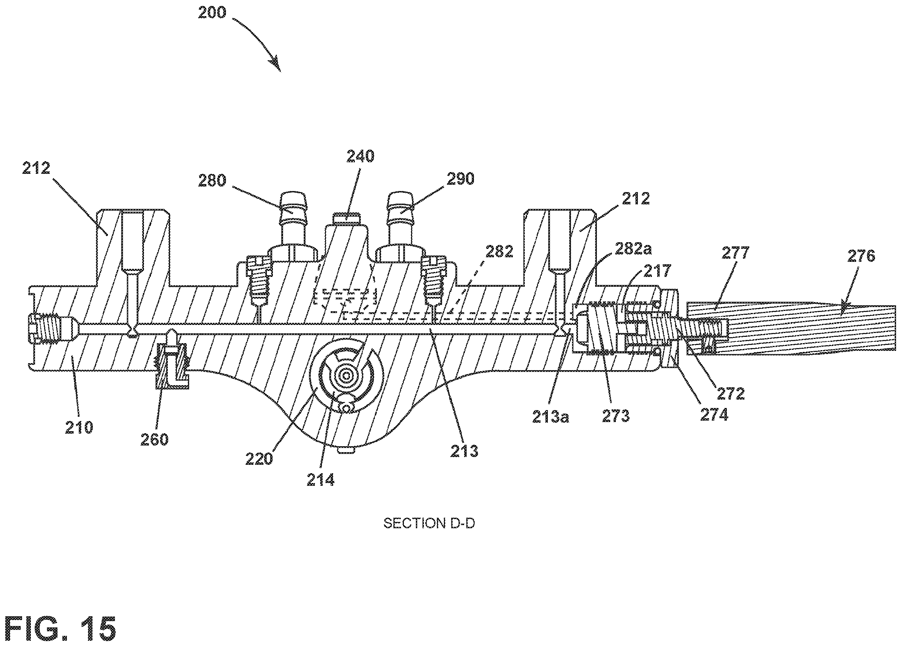

FIG. 15 is a cross-sectional view of the integrated manifold system of FIG. 13 along section line D-D.

### DETAILED DESCRIPTION OF THE PREFERRED EMBODIMENTS

The invention summarized above may be better understood by referring to the following description, claims, and accompanying drawings. This description of an embodiment, set out below to enable one to practice an implementation of the invention, is not intended to limit the preferred embodiment, but to serve as a particular example thereof. Those skilled in the art should appreciate that they may readily use the conception and specific embodiments disclosed as a basis for modifying or designing other methods and systems for carrying out the same purposes of the present invention. Those skilled in the art should also realize that such equivalent assemblies do not depart from the spirit and scope of the invention in its broadest form.

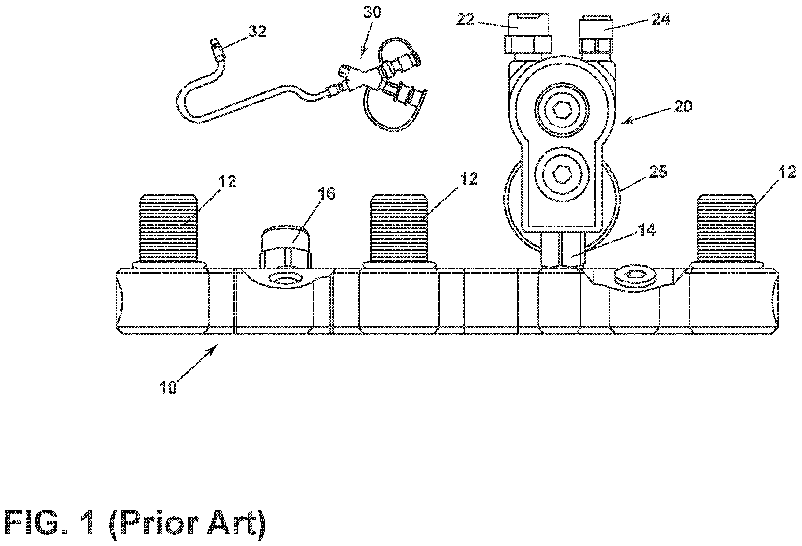

FIG. 1 shows a prior art manifold system 10 for connecting high pressure air bottles (not shown) to a first stage regulator 20, which first stage regulator 20 in turn delivers air to a second stage regulator on an operator's mask (not shown), such as a gas mask. Manifold 10 includes bottle ports 12 that removably receive high pressure air bottles or canisters, which deliver air through an internal port to a regulator adapter 14 that removably receives a first stage regulator 20. First stage regulator 20 typically includes a low pressure hose attachment 22 for delivering air pressure through a hose to the operator's mask, and a low pressure relief valve 24 to allow an operator to regulate the amount of pressure delivered to their mask and/or to allow air exceeding a desired pressure to be bled from the regulator 20. First stage regulator 20 also typically includes an on/off valve 25 that controls delivery of air from regulator adapter 14 into first stage regulator 20. Manifold 10 typically includes a high pressure relief valve 16 that is attached to manifold 10 with yet another separate fitting to allow emergency release of air pressure from the system. Moreover, in order to recharge canisters attached to manifold 10, a separate recharge assembly 30 must be provided and carried by the operator. Such recharge assembly 30 includes a hose fitting 32 that must be fitted to yet another fitting (not shown) on manifold 10 in order to allow refill of air canisters attached to manifold 10 through cylinder ports 12.

Such prior art assemblies have disadvantages for the operator, particularly when working in high-stress environments, as well as for the manufacturer or supplier of such systems. The various adapters necessary for attachment of the first stage regulator 20, the high pressure relief valve 16, and the recharge port 30 all create potential points of failure, and overall add weight to the system that the operator must carry. Moreover, such varied components add unnecessary complexity to the system which increases costs in materials and manufacturing.

FIGS. 2, 3, and 4 show top perspective, front, and back views, respectively, of an improved manifold system 100 in accordance with certain aspects of an embodiment of the invention, which integrates a first stage regulator 120, a male quick disconnect 140 for recharging bottles attached to manifold system 100 and for providing an alternative source of air to manifold system 100, a high pressure relief valve 160, an on/off valve 170, a low pressure hose attachment 180, and a low pressure relief valve 190 all in a single, unitary manifold body 110. Such assembly significantly improves upon previously known assemblies by dramatically decreasing the number of adapter and integration fittings required for assembly of the foregoing components, and by making the entire assembly lighter and smaller (and thus of less physical load on the operator), while likewise improving quality control and assembly time by having fewer potential points of failure.

With continued reference to FIGS. 2-4, the top view of manifold system 100 of FIG. 5, and the various cross-sectional views of manifold system 100 of FIGS. 6-9, manifold body 110 is a single, unitary (i.e., one piece) assembly. In a particularly preferred embodiment, manifold body 110 may be milled out of billet aluminum. In other configurations, such as when manifold 100 is intended for use in the pressure-regulated delivery of high pressure oxygen, manifold 100 may be milled out of brass, titanium, or such other materials as may be best suited to a particular application, as may be selected by persons skilled in the art. Manifold body 110 includes bottle ports 112 that removably receive readily commercially available air cylinders (not shown) of standard configuration, which cylinders are available from a variety of manufactures. With particular reference to the cross-sectional view of FIG. 7*a* (along section line A-A of FIG. 6) showing manifold body 110 with all other elements removed, manifold body 110 is milled to form a first stage regulator chamber 114 extending from a front side 110 *a* of manifold body 110, through manifold body 110 and to a back side 110 *b* of manifold body 110, which first stage regulator chamber 114 is shaped to receive first stage regulator 120. Manifold body 110 is further milled to form a quick disconnect fitting port 115 configured to removably receive, such as by way of a threaded connection, male quick disconnect 140, and a high pressure feed port 116 that fluidly communicates quick disconnect fitting port with 115 with first stage regulator chamber 114. Still further, manifold body 110 is milled to form on/off valve receiver 117 configured to removably receive, such as by way of a threaded connection, on/off valve 170. On/off valve receiver 117 is in fluid communication with high pressure air channel 113, such that (as discussed in greater detail below and with reference to FIG. 10) on/off valve 170 in on/off valve receiver 117 is operative to control air flow from high pressure air channel 113 into a head space 117*a* (FIG. 6) at the top end of on/off valve receiver 117. Head space 117*a* of on/off valve receiver 117 is also in fluid communication with quick disconnect fitting port 115 via inlet 115*a*, such that when on/off valve 170 is open, air flows from air bottles attached to bottle ports 112, through high pressure air channel 113, into head space 117*a* of on/off valve receiver 117, and out of head space 117*a* through inlet 115*a* into quick disconnect fitting port 115. From quick disconnect fitting port 115, high pressure air is then delivered through high pressure feed port 116 into first stage regulator chamber 114, and as discussed in greater detail below, is ultimately delivered from first stage regulator chamber 114 through outlet channel 118 (FIG. 8) which is likewise milled into manifold body 110. An outer end of outlet channel 118 is configured to receive low pressure hose attachment 180 for delivering air from manifold system 100 to a user's mask. Preferably, a relief valve channel 119 is also milled into manifold body 110 and is similarly in communication with first stage regulator chamber 114, and receives low pressure relief valve 190 at an outer end of relief valve channel 119.

As mentioned above, and with particular reference to FIGS. 6 and 9 and the exploded view of FIG. 10, on/off valve 170 controls the flow of air from high pressure air channel 113 to quick disconnect fitting port 115. In that regard, on/off valve 170 includes a manually operable hand wheel 171, a valve stem 172, and a plug 173 engaged by valve stem 172 to open and close port 113*a* that communicates high pressure air channel 113 with head space 117*a*. Plug 173 is biased by spring 174 (the biasing force of which is adjustable via nut 175) towards closure of port 113 *a*, with the closing force being adjustable by turning hand wheel 171 so as to allow pressurized air to flow from high pressure air channel 113, into head space 117*a*, and through inlet 115*a* to quick disconnect fitting port 115. In certain embodiments, on/off valve receiver 117 preferably forms a downwardly angled opening in manifold body 110, which positions hand wheel 171 for access by an operator from the underside of manifold body 110, extending downward and to one side of manifold body 110 to allow easy access to an operator when reaching around their body to control the manifold system 100, without requiring the operator to remove the system from their back to turn the system on and off.

Thus, when on/off valve 170 is opened, full bottle pressure is introduced from bottle ports 112 into air channel 113, to head space 117*a*, through inlet 115*a* into quick disconnect fitting port 15, and from disconnect fitting port 15 through high pressure feed port 121 into first stage regulator 120.

With continued reference to FIGS. 6, 9, and 10, high pressure relief valve 160 is provided in an underside of manifold body 110. High pressure relief valve 160 includes a burst plug 161 and a burst safety disc 162 in fluid communication with high pressure air channel 113. In certain embodiments, high pressure relief valve receiver 163 is milled into a bottom face of manifold body 110 on an opposite side of first stage regulator 120 from on/off valve receiver 117 and extends vertically from the bottom face of manifold body 110 into high pressure air channel 113, and removably receives (such as by way of non-limiting example via a threaded connection) high pressure relief valve 160 therein. This configuration positions high pressure relief valve 160 largely within the profile of manifold body 110 so as to avoid unnecessary obstructions on the exterior of manifold system 100. In the event of an overpressure condition occurring in air bottles attached to bottle ports 112 and/or in high pressure air channel 113, high pressure relief valve 160 will burst and allow air within high pressure air channel 113 to escape from the bottom of manifold body 110 to atmosphere, thus avoiding the risk of inadvertently delivering over-pressure air into regulator 120, which could damage the components of regulator 120. Importantly, high pressure relief valve receiver 163, and thus high pressure relief valve 160 itself, is pressurized at any time that air bottles connected to manifold system 100 see pressure, thus ensuring that the pressure relief valve 160 cannot be bypassed. Likewise, the downward vertical orientation of high pressure relief valve receiver 163 ensures that in the event that such pressure relief valve 160 bursts, the escaping high pressure air stream is directed downward so as to not cause injury to the operator or damage to other operator-worn equipment.

Preferably blanking plugs 101 are removably positioned in manifold body 110 at opposite ends of high pressure air channel 113 which, when fully installed, seal air channel 113. Such blanking plugs 101 may provide access to air channel 113, thus allowing additional components (sensors, etc.) to be added to manifold system 100.

With continued reference to FIGS. 6, 9, and 10, male quick disconnect 140 may be removably positioned (such as, by way of non-limiting example, through a threaded connection) within quick disconnect fitting port 115. Quick disconnect fitting port 115 is milled into manifold body 110 at a downward angle and in line with an imaginary axis extending through first stage regulator 120, which positions quick disconnect 140 so that its inlet points upward and away from manifold body 110 towards the rear side of manifold body 110. This configuration allows easy connection of a high pressure air supply without interfering with additional equipment that may be carried on the operator's back.

Quick disconnect 140 includes a fitting 141 configured to receive a quick connect adapter from a hose that may supply high pressure air to manifold system 100, and includes a check valve (not shown) of standard configuration to allow airflow into quick disconnect fitting port while preventing air from escaping in the opposite direction. Quick disconnect 140 is sized such that when it is positioned in quick disconnect fitting port 115, the base 142 of quick disconnect 140 is spaced apart from an inner wall of disconnect fitting port 115 so as to define a head space 115b, which as explained above receives high pressure air through inlet 115*a* (FIG. 7*a*)*,* and delivers air to first stage regulator 120 through high pressure feed port 116. In the event that on/off valve 170 is closed, such that no air is delivered to head space 115*b* from inlet 115*a* (and thus from bottles attached to bottle ports 112), air may still be provided into first stage regulator by connecting a source of high pressure air to male quick disconnect 140. In this configuration, the external source of air connected to male quick disconnect 140 may allow the operator to use manifold system 100 with such source of air delivering breathable air through first stage regulator 120 instead of air from bottles attached at bottle ports 112, thus allowing air within those bottles to be saved when desired, and/or provide air when such bottle supply is depleted. Likewise, when an external source of air is connected to male quick disconnect 140 and on/off valve is opened, air may flow through quick disconnect 140 into head space 115*b*, out through inlet 115*a* and into high pressure air channel 113, and from high pressure air channel 113 into bottles attached to bottle ports 112 for purposes of refilling those bottles with usable air, all without requiring attachment of a separate recharge assembly 30 (FIG. 1) as required by previously known systems.

Optionally and if desired for certain operations, an adapter (not shown) may alternatively be provided having a bottom portion configured identical to the bottom portion of quick disconnect 140 for removable fitment within quick disconnect fitting port 115, which adapter may then connect, such as by way of a hose, to a remote quick disconnect fitting 140 (such as where a refilling air supply is positioned remotely from an operator that is using the improved manifold system 100).

Still further, and as best viewed in FIG. 7, an auxiliary gauge port 115 c is preferably milled into manifold body 110 from a top face of manifold body 110 so as to be in fluid communication with head space 115 *b.* Gauge port 115 c may removably receive a gauge (not shown), such as a pressure gauge, through (by way of non-limiting example) a threaded connection. Through positioning of such auxiliary gauge port 115 c in direct fluid communication with head space 115 *b,* a pressure reading is provided that shows actual pressure that is being supplied through high pressure feed port 116 into first stage regulator 120, as opposed to merely providing current air pressure within bottles attached to bottle ports 112. This configuration ensures that the operator has a correct indicator of pressure being supplied to first stage regulator 120 regardless of the condition of the bottles attached to bottle ports 112. In the event that a pressure gauge is not required or desired for a given operation, it may be removed from auxiliary gauge port 115 c and a removable plug may be placed in auxiliary gauge port 115 *c*.

As explained above, and with particular reference to FIGS. 7, 9, and 10, first stage regulator 120 extends through first stage regulator chamber 114 from a front side 110 *a* of manifold body 110 to a back side 110 *b* of manifold body 110. The portion of regulator 120 that is nearest the back side 110 *b* of manifold body 110 comprises the high pressure portion of regulator 120, while the portion that is nearest the front side 110 *a* of manifold body 110 comprises the low pressure portion of regulator 120 that feeds air outlet channel 118. Thus, the high pressure side of regulator 120 includes a balance plug 122 at an outer-most portion of regulator 120, a valve 123, and a valve spring 124. A diaphragm lifter 125 extends through a seat support 126 and orifice seat 127 into a head end of valve 123, and at an opposite end engages a diaphragm 128 that controls air flow from first stage regulator chamber 114 of manifold body 110 into outlet channel 118 (and thus to low pressure hose attachment 180). Diaphragm 128 is held against a seat formed by first stage regulator chamber 114 by a diaphragm clamp ring 129, and is biased toward a closed, sealing position by spring 130 and spring carrier 131, the biasing force of which may be adjusted by spring adjuster 132.

As shown in FIG. 12 depicting manifold system 100 with air cylinders 196 positioned thereon, manifold body 110 preferably has a total length dimension that is less than the total width of the air cylinders 196 and a carrier 198 that holds cylinders 196, thus significantly reducing the profile of manifold 100 and allowing far greater maneuverability of the operator over previously known configurations.

As mentioned above, manifold system 100 includes low pressure hose attachment 180 removably attached to outlet channel 118 in manifold body 110 and configured for attachment to a hose to deliver breathable air from the low pressure outlet side of first stage regulator 120 to, by way of non-limiting example, a second stage regulator on an operator's mask. Low pressure hose attachment 180 is positioned to one side of male quick disconnect 140. A quick disconnect fitting of standard configuration may be included on low pressure hose attachment 180, allowing quick connect and quick disconnect of a supply hose (not shown) that, in turn, ultimately delivers breathable air to the operator through, by way of non-limiting example, a respirator mask.

Similarly, low pressure relief valve 190 is configured to allow excess pressure in the low pressure outlet side of first stage regulator 120 to automatically be bled from the low pressure side of first stage regulator 120. Low pressure relief valve 190 is attached to relief valve channel 119 in manifold body 110, which relief valve channel 119 is in fluid communication with the low pressure outlet side of first stage regulator 120. Low pressure relief valve 190 is positioned to a second side of male quick disconnect 140 opposite low pressure hose attachment 180.

Referring now to FIG. 13, another integrated manifold system 200 is illustrated in accordance with various aspects described herein. The manifold system 200 is similar to the manifold system 100. Therefore, like parts will be described with like numbers increased by 100. It will be understood that descriptions or functions of the like parts of the manifold system 100 also apply to the manifold system 200, except where noted.

The manifold system 200 includes a manifold body 210 having bottle ports 212, a first stage regulator 220, a high pressure relief valve 260 (visible in FIG. 14), an on/off valve 270, a low pressure hose attachment 280, and a low pressure relief valve 290. The manifold body 210 can also include a male quick disconnect 240 for recharging bottles attached to manifold system 200 or for providing an alternative source of air to manifold system 200. Furthermore, a high pressure air channel 213 (visible in FIG. 14) extends through the manifold body 210 and fluidly couples the bottle ports 212, high pressure relief valve 260, low pressure relief valve 290, first stage regulator 220, and on/off valve 270. In this manner air can flow into the first stage regulator 220 via the bottle ports 212 or the male quick disconnect 240.

One difference compared to the manifold system 100 is that the on/off valve 270 is positioned on a side of the manifold body 210 as shown. Another difference is that the manifold system 200 includes a manually-operated hand wheel 271 that is positioned remotely from the manifold body 210. A shaft 276 is provided having a first end 277 coupled to the on/off valve 270 and a second end 278 coupled to the hand wheel 271. The shaft 276 can be configured to transfer rotary motion such that rotation of the hand wheel 271 at the second end 278 can effect rotation of the on/off valve 270 at the first end 277 of the shaft 276. In the example shown, the shaft 276 is in the form of a flexible shaft. In another non-limiting example, the shaft 276 can be in the form of a rigid shaft extending away from the manifold body 210.

Turning to FIG. 14, an exploded view of the manifold body 210 is illustrated. A high pressure relief valve 260 is provided and includes a burst plug 261 and a burst safety disc 262 in fluid communication with high pressure air channel 113 (visible in FIG. 15). The on/off valve 270 includes a valve stem 272, a plug 273, and a bonnet 274 engaged by valve stem 272 to open and close at least one port within the manifold body 210. In addition, manifold body 110 can be milled to form a first stage regulator chamber 214 extending from a front side 210a of manifold body 210, through manifold body 210 and to a back side 210b of manifold body 210, which first stage regulator chamber 214 is shaped to receive first stage regulator 220.

Another difference compared to the manifold system 100 is that the first stage regulator 220 includes a high pressure inlet jet 284 and an inlet jet cap 285 near the back side 210b of the manifold body and at an outer-most portion of the regulator 220. Still another difference is that the first stage regulator 220 includes an inner spring 286, an outer spring 287, a piston seat assembly 288, and a piston cap 289 near the front side 210a of the manifold body 210.

The piston seat assembly 288 can extend into the manifold body 210 and through a sealed chamber to fluidly communicate low pressure air to portions of the manifold body 210, such as an outlet channel 218. More specifically, the piston seat assembly 288 can engage the high pressure inlet jet 284 to control air flow from first stage regulator chamber 214 into outlet channel 218 and to the low pressure hose attachment 280. For instance, the large end of the piston seat assembly 288 can engage an internal face of the piston cap 289. When assembled, the piston cap 289 engages with the front side 210a of the manifold body 210 to form a seal, thereby maintaining pressure within the chamber. The inner spring 286 and outer spring 287 bias the piston seat assembly 288 to an open position toward the front side 210a of the manifold body 210. It is contemplated that the inner spring 286 and outer spring 287 can form a redundancy for the manifold system 200, such that the inner spring 286 or outer spring 287 alone can bias the piston seat assembly 288 to a desired position during operation.

Referring now to FIG. 15, a cross-sectional view of the manifold system 200 is illustrated along line D-D. The manifold body 210 includes an on/off valve receiver 217 located on a side of the manifold body 210 in fluid communication with the high pressure air channel 213. The on/off valve receiver 217 is configured to removably receive, such as by way of a threaded connection, the on/off valve 270. The on/off valve 270 controls the flow of air from high pressure air channel 213 to the male quick disconnect 240.

Still another difference compared to the manifold system 100 is that multiple air channels can be provided within the manifold body 210. In the example shown, a second air channel 282 is illustrated parallel to the high pressure air channel 213 and fluidly coupled to the on/off valve 270 via a port 282a. The second air channel 282 can be utilized for suppling high pressure air or low pressure air. In the example shown the second air channel 282 fluidly couples the on/off valve 270 to the male quick disconnect 240. Any number of air channels are contemplated.

The on/off valve 270 can open and close at least one port, including port 213a and port 282a. In one example, plug 273 can be biased towards closure of port 213a or 282a, with the closing force being adjustable by turning hand wheel 271 (FIG. 13) so as to allow pressurized air to flow from high pressure air channel 213 or second air channel 282 to the quick disconnect 240. The first end 277 of the shaft 276 can be configured to couple with, or be made integral with, the bonnet 274 for operatively coupling to the plug 273.

With general reference to FIGS. 13-15, during operation, the second end 278 of the flexible shaft 276 can be routed to a position conveniently reachable by a user. In one example wherein the manifold body 210 is positioned near the user's back, the second end 278 of the flexible shaft 276 can be routed around the user's waist, over the user's shoulder, or otherwise directed to the user's front side. The user can rotate the remotely-positioned hand wheel 271, and the shaft 276 can transfer rotary motion of the hand wheel 271 from the second end 278 to the first end 277. In this manner, rotation of the hand wheel 271 can effect rotation of at least one of the bonnet 274 or plug 273 to open or close the port 213a or the port 282a. For instance, the second air channel 282 can supply high pressure air to or from the quick disconnect 240 if the plug 273 is manually opened, including by rotation of the hand wheel 271.

The combination disclosed herein of a single, compact, unitary assembly of a manifold body for connecting air supply bottles with a first stage regulator is a significant improvement over previously known systems, as it reduces potential points of failure in the system, reduces overall weight of the system, and eases the burden on the operator by not requiring them to carry and keep track of multiple, separate components for these features. Further beneficial features of the manifold system described herein likewise include the addition of a male quick disconnect 140, 240 enabling cylinder recharge and alternative air supply to that provided by the bottles, an on/off valve 170, 270, and pressure safety devices again all in a single, compact assembly. Previously known systems have typically required separate assemblies with various flow lines among them to achieve these varied functions of a respirator system, and such dispersed components have added size and weight to those systems that make it difficult for the user to operate, particularly in stressful environments. Those skilled in the art will appreciate that a system configured in accordance with the invention will significantly reduce the stress experienced by an operator in a dangerous environment, while ensuring that they have ready and continuous access to a reliable breathable air delivery system.

Having now fully set forth the preferred embodiments and certain modifications of the concept underlying the present invention, various other embodiments as well as certain variations and modifications of the embodiments herein shown and described will obviously occur to those skilled in the art upon becoming familiar with said underlying concept. By way of non-limiting example, and with reference to FIGS. 11 and 13, manifold body 110, 210 may include more than 2 bottle ports 112, 212 by simply extending the length of manifold body 110, 210 and air channel 113, 213 that interconnects bottle ports 112, 212. Similarly, only one bottle port 112, 212 may be provided to further limit the physical profile of the manifold system 100, 200. It should be understood, therefore, that the invention may be practiced otherwise than as specifically set forth herein.

## Claims

1. An air supply system comprising:
a one-piece manifold body (110, 210) having:
at least one bottle port (112, 212) configured to removably receive a bottle of pressurized air;
a first stage regulator chamber (114, 214);
a quick disconnect fitting port (115, 215);
a first air channel (113, 213) in fluid communication with the at least one bottle port (112, 212) and the first stage regulator chamber (114, 214);
an outlet channel (118, 218) in fluid communication with the first stage regulator chamber (114, 214); and
an on/off valve (170, 270) operatively positioned on a side of the manifold body (110, 210) and engaging the first air channel (113, 213) to regulate air flow between the at least one bottle port (112, 212) and the first stage regulator chamber (114, 214).

2. The air supply system of claim 1, further comprising a first stage regulator (120, 220) operatively engaging the first stage regulator chamber (114, 214).

3. The air supply system of claim 2, wherein the first stage regulator (120, 220) extends through the first stage regulator chamber (114, 214) from a front side (110a, 210a) of the manifold body (110, 210) to a back side (110b, 210b) of the manifold body (110, 210) opposite the front side.

4. The air supply system of claim 1, further comprising a mask hose quick disconnect (180, 280) in fluid communication with the outlet channel (118, 218) and configured to removably receive an air hose.

5. The air supply system of claim 1 further comprising a manually-operated hand wheel (171, 271) positioned remotely from the manifold body (110, 210).

6. The air supply system of claim 5 further comprising a shaft (276) having a first end connected to the hand wheel (171, 271) and a second end connected to the on/off valve (170, 270).

7. The air supply system of claim 1 further comprising a high-pressure inlet jet (284) and an inlet jet cap (285) near a back side of the manifold body.

8. The air supply system of claim 1 further comprising an inner spring (286), an outer spring (287), a piston seat assembly (288), and a piston cap (289) near a front side (110a, 210a) of the manifold body.

9. The air supply system of claim 1 further comprising multiple air channels (113, 213, 282) within the manifold body.

10. The air supply system of claim 9 wherein a second air channel (282) is disposed generally parallel to the first air channel (113, 213) and fluidly couples to the on/off valve (170, 270) via a port (140, 240).

11. The air supply system of claim 9 wherein a second air channel (282) supplies high pressure air or low pressure air to the on/off valve (170, 270) via a male quick disconnect (140, 240).

12. The air supply system of claim 1, wherein the on/off valve (170, 270) extends angularly outward from the one-piece manifold body (110, 210) at a non-vertical angle.

13. The air supply system of claim 1, further comprising a quick disconnect fitting (140, 240) removably mounted in a quick disconnect fitting port (115, 215) and configured for connection to an air supply.
